# EUROPEAN PATENT SPECIFICATION

(11) **EP 0 519 572 B1**
(45) Date of publication and mention of the grant of the patent: **04.09.1996**
(21) Application number: 92201797.5
(22) Date of filing: 18.06.1992
(51) Int. Cl.: A61H 33/00, A61H 33/02, A61L 2/18, B08B 9/00

(54) **Method, apparatus and assembly of parts for cleaning a pumpsystem for causing air or bathwater to flow out forcefully into a bath**
Verfahren, Vorrichtung sowie Zusammenstellung von Teilen zum Reinigen sowie Pumpsystems zum Einbringen von Luft oder Wasser in ein Bad
Méthode, dispositif et assemblage de pièces pour nettoyer un système de pompage pour l'introduction forcée d'air ou d'eau dans un bain

(30) Priority: 20.06.1991 NL 9101068
(43) Date of publication of application: 23.12.1992
(73) Proprietor: SANILUX B.V., NL-9665 BB Oude Pekela (NL)
(72) Inventor: Leeuwerik, Berend c/o Sanilux B.V., NL-9665 BB Oude Pekela (NL)
(74) Representative: Smulders, Theodorus A.H.J., Ir.

(56) References cited:
- EP-A- 0 059 407
- EP-A- 0 215 514
- EP-A- 0 338 607
- EP-A- 0 396 117
- FR-A- 2 568 264
- GB-A- 1 229 582

## Description

This invention relates to a method as set forth in the preamble of claim 1. Such a method is known from EP-A-0 396 117. The invention also relates to an apparatus according to the preamble of claim 8. Such an apparatus is known from EP-A-0 215 514.

In baths that are generally referred to as "whirlpools", a pump system is used. Because dirt will deposit in such a pump system and thereby constitute a breeding ground for bacteria, fungi and algae, it is good practice, for reasons of hygiene, to clean and disinfect such pump systems after each use by circulating water with a cleaning agent dissolved therein through the pump system. This is typically effected by filling the bath after use with a certain amount of this solution and circulating it through the system.

It has been found that especially in regions where the mains water has a high lime content, lime adheres to the piping and soap residues and the like in turn adhere to the lime. Such layers adhering to the lime form a good breeding ground for germs and algae.

In the regions referred to it is problematic to maintain adequate hygienic conditions in baths of the type referred to even if cleaning is done with an increased concentration of cleaning agent and a cleaning agent with decalcifying properties is used. An additional drawback is that these last-mentioned measures entail an increased burdening of the environment.

The object of the invention is to provide a method of the type described in the preamble, which provides for improved cleaning of a pump system while keeping the burden on the environment relatively low.

This object is realized according to the present invention in that in a method as described in the preamble the characteristic features according to claim 1 are employed.

Inasmuch as decalcification and disinfection are effected separately, a decalcifier can be selected without taking into consideration its interaction (such as the formation of salt crystals) with disinfectant and vice versa. Thus, in the first place a particularly effective removal of the lime deposits can be achieved, so that a lesser disinfectant activity will suffice, and subsequently a particularly effective disinfection can be achieved.

As a disinfectant, it is for instance possible to select an agent that does not eliminate amoebas such as are used in sewage treatment plants.

The method according to the invention can be used for a conventional bath of the type described hereinabove, provided the decalcifiers and disinfectants referred to are at hand. Hygiene can be maintained in a particularly reliable manner when an apparatus adapted for the method is used for carrying out the method.

The invention can also be embodied, in an apparatus of the above-identified type, which is specifically adapted for carrying out the method according to the invention as defined in claim 1 by incorporating the characterizing features of claim 8.

Hereinafter, the invention will be further illustrated and explained on the basis of one embodiment, with reference to the accompanying drawings, in which:
Fig. 1 is a sectional side elevation of a bath fitted with an apparatus according to the invention, and
Fig. 2 is a diagram of an apparatus according to the present invention.

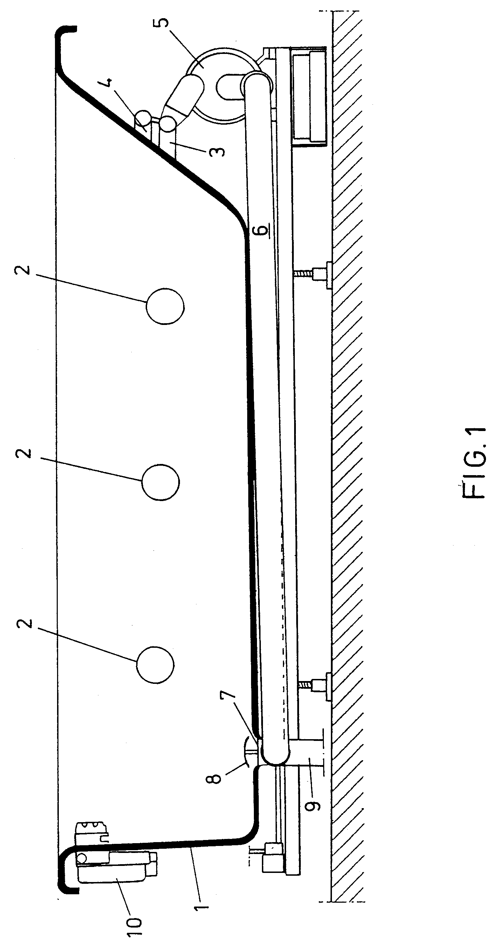

Fig. 1 shows a bath designed for use of the method according to the invention.

The bath comprises a bathtub 1 provided with outlet openings 2 for causing air or bath water to flow out forcefully under water. Connected to the openings 2 are pipes 3, 4 which are in communication with a pump 5. The pump 5 in turn communicates with a supply pipe 6 connecting to a drain opening 7 in the bottom of the bathtub. The pump 5 and the pipes 3, 4, 6 form the pumping system for circulating bath water or air. Of course, in the case where only air is pumped, the pipe 6 is not used. Air can be drawn in via a supply opening (not shown) upstream of the pump 5.

The drain opening 7 can be shut off by a valve 8 if the bath is to be used without the water flowing through the openings 2. Connecting to the bath is a discharge channel 9 in which is arranged a discharge valve (not shown). By operating this discharge valve, the bath can be drained.

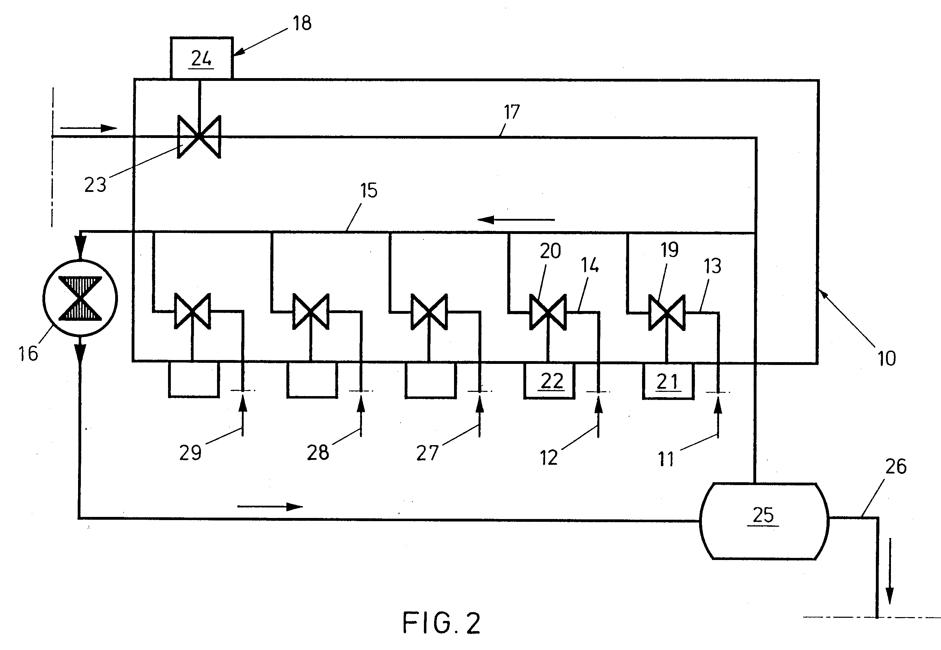

For the method according to the invention to be carried out in a simple manner, the bath is fitted with an apparatus of which a part is schematically shown in Fig. 2. This part is accommodated in the housing indicated by the reference numeral 10 in Fig. 1.

This apparatus comprises two reservoirs represented by the feed arrows 11 and 12 for decalcifier and disinfectant, respectively. These reservoirs 11 and 12 communicate with connecting pipes 13 and 14, respectively, and can be connected selectively with a supply pipe 15. The apparatus further comprises an element 16 for dosaging liquid supplied from each of the reservoirs 11 and 12, and a water supply pipe 17 comprising dosaging means 18. For the selective connection of the reservoirs 11 and 12 with the supply pipe 15, valves 19 and 20 have been arranged in the supply pipes 13 and 14, respectively. Valves 19 and 20 can be operated by electromagnets 21 and 22, respectively. The dosaging means 18 comprise a valve 23 and an electromagnet 24 for operating valve 23. The supply pipe 15 and the water supply pipe 17 open into a mixing chamber 25. A discharge pipe 26 extends from the mixing chamber to the bathtub 1 (Fig. 1).

In the method according to the invention, during a first cleaning cycle a decalcifier is used as cleaning agent and during a subsequent cleaning cycle a disinfectant is used as cleaning agent. Depending on the hardness of the water, it is possible, for instance, to use decalcifier and disinfectant alternately or to use a disinfectant twice or more often in succession before decalcifier is used again. Preferably, each time the pumping system has been used, a complete cleaning cycle is run.

For decalcifier or disinfectant to be supplied from the reservoirs 11 and 12, respectively, valves 19 and 20, respectively, are opened and the dosaging element 16 is actuated. This dosaging element is designed according to the present embodiment as a pump which draws the liquid from the reservoirs 11, 12 of which the valve 19, 20, respectively, is opened. Depending on the number of strokes or revolutions or the duration of the operation of the pump 16, the dose of the agent in question is determined.

The dosaging element 16 is arranged in the supply pipe 15 and both ends of the supply pipe 15 are connected to the water supply pipe 17. This offers the advantage that by actuating the dosaging element 16 when water is being supplied, clean water flows through the supply pipe 15 so that it will be rinsed clean. Thus, for the dosaging of both disinfectant and decalcifier, a single dosaging element 16 can be used without residues of one agent being supplied along with the other agent.

To provide that the hygiene in the pumping system can be maintained in a reliable manner, it is preferred that the termination of the use of the bath be detected and, in response thereto, at least one complete cleaning cycle be carried out. The detection of the termination of the use of the bath can for instance be detected through the operation of the discharge valve. A cleaning cycle can then be automatically started and carried out after, for instance, 10 minutes.

The dosaging of the amount of cleaning agent is preferably controlled depending on data stored in a data processor. Thus, the dosaging can simply be adjusted, for instance to the hardness of the water, the type of decalcifier or disinfectant that is being used, the type of bath and the type of pumping system.

For the dosaging of the amount of cleaning agent to be controlled depending on data stored in a data processor and for a cleaning cycle to be carried out automatically, the apparatus according to the invention is preferably provided with a data processor with connections for means 19, 20 associated with a particular reservoir 11, 12 for connecting that reservoir 11, 12 to the supply pipe 15, the dosaging element 16, means for operating a discharge valve, means for operating the pump 5 of the pumping system and the dosaging means 18 of the water supply 17.

The data are preferably stored in the data processor from a universal data processor. Thus, the data processor of the apparatus according to the invention can be of simple design.

A particularly quick adaptation of the data in the data processor of the apparatus is possible if the data are inputted to the universal data processor via a telephone line and a modem. It is then possible, for instance, that the user of a bath reports data regarding changed conditions or the use of a different disinfectant or decalcifier to the supplier, which supplier, on the basis of the most recent data, compiles an adapted set of data for the data processor of the apparatus and inputs these data to the universal data processor of the user via the modem. The user in turn inputs the modified set of data to the data processor of the apparatus. The universal data processor can for instance be a data processor of a commonly used PC.

For inputting data to the data processor from a universal data processor, the data processor of the apparatus may be provided with a storage module and a connecting gate for its connection to a universal data processor.

When a cleaning cycle has been completed, the pumping system is preferably substantially drained.

To that end, according to the present invention, the bath with an apparatus according to the invention further comprises a pumping system 3, 4, 5, 6 that is self-draining.

After draining, sufficient disinfectant is left behind to prevent germination. If a suitable disinfectant is used, this will even be the case if the pumping system fills up and drains again, as is the case when a bath is taken without the pumping system being actuated. Draining of the pumping system further prevents the precipitation of dirt contained in water that is present in the pumping system.

Preferably, the apparatus comprises at least a third reservoir (arrow 27). The apparatus according to the present embodiment further comprises a fourth and a fifth reservoir represented by arrows 28 and 29.

This makes it possible to further use the apparatus for dosaged supply of bathing oil, for instance. In particular when medicinal oils are used, accurate dosaging is important. As with the disinfectant and the decalcifier, it is advantageous if dosaging can be effected on the basis of data stored in a data processor. The design of the apparatus shown in Fig. 2 ensures that no residues of disinfectant or decalcifier are carried along when bathing oil or the like is being supplied from one of the reservoirs 27-29.

The reservoirs are arranged on that side of the wall of the bathtub 1 that faces away from the user, so that they are hidden from view. This is desirable because in particular reservoirs filled with bathing oil can in the course of time acquire an unattractive appearance without this being any indication of the quality of the bathing oil. A further advantage of arranging the reservoirs behind the wall of the tub 1 is that the reservoirs are thus shielded from light, which has a negative influence on the storage life of ethereal oils.

A disinfectant that is particularly suitable for use in the present invention is the disinfectant with German admission number UBA-07700909. A decalcifier which is particularly suitable for use in the present invention is the decalcifier with German admission number UBA-07700059. Depending on the legislation in force, however, it may be necessary to choose other agents.

## Claims

1. A method of cleaning a bath comprising a pumping system (3, 4, 5, 6) for causing air or bath water to flow out forcefully into a bathtub (1), in which method water and cleaning agents dissolved therein are circulated through said pumping system (3, 4, 5, 6), characterized in that during a first cleaning cycle a decalcifier is used as cleaning agent and during a subsequent cleaning cycle a disinfectant is used as cleaning agent.

2. A method according to claim 1, characterized in that the relative frequency of the use of decalcifier as cleaning agent depends on the hardness of the water.

3. A method according to claim 1 or 2, characterized in that the termination of the use of the bath is detected and, in response thereto, automatically at least one cleaning cycle is carried out.

4. A method according to claim 3, characterized in that the dosaging of the amount of cleaning agent is controlled depending on data stored in a data processor.

5. A method according to claim 4, characterized in that the data are stored in the data processor from a universal data processor.

6. A method according to claim 4, characterized in that the data are inputted to the universal data processor via a telephone line and a modem.

7. A method according to any one of the preceding claims, characterized in that upon completion of a cleaning cycle the pumping system (3, 4, 5, 6) is substantially drained.

8. A bath comprising
a bathtub (1),
a pumping system (3, 4, 5, 6) including a pump (5) for causing air or bath water to flow out forcefully into the bathtub (1),
means for operating the pump (5),
a supply pipe (15) communicating with a discharge pipe (26) opening into the bathtub (1),
at least two reservoirs (11, 12),
one of said reservoirs for containing a disinfectant,
means 19, 20) for selectively connecting each of said reservoirs (11, 12) with the supply pipe (15),
at least one liquid dosaging element (16) for dosaging liquid supplied from each of the reservoirs (11, 12),
a clean water supply pipe (17) with dosaging means (18),
a discharge channel (9) for draining the bath,
a discharge valve arranged in said discharge channel (9),
means for operating said discharge valve, and
a data processor operatively connected for operating the pump (5), the liquid dosaging element (16), and the means (19, 20) for selectively connecting each of the reservoirs (11, 12) with the supply pipe (15),
characterized in that
the other one of said reservoirs (11) is a reservoir for containing a decalcifier,
the data processor is also operatively connected to the dosaging means (18) of the clean water supply pipe (17) and to the means for operating said discharge valve arranged in said discharge channel (9) for draining the bath and in that
the data processor is programmed for operating the dosaging means (18) of the clean water supply pipe (17) and the means (19, 20) for selectively connecting each of said reservoirs (11, 12) with the supply pipe (15) such that during a first cleaning cycle clean water to be circulated through the pumping system (3, 4, 5, 6) is supplied and liquid from a reservoir (11) for decalcifier is mixed with said water and during a second cleaning cycle clean water to be circulated through the pump system (3, 4, 5, 6) is supplied and liquid from a reservoir (12) for disinfectant is mixed with said water.

9. A bath according to claim 8, characterized in that the dosaging element (16) is arranged in the supply pipe (15) and a downstream end of the supply pipe (15) communicates with a discharge pipe (26) opening into the bathtub (1).

10. A bath according to claim 8 or 9, characterized by at least a third reservoir (arrow 27).

11. A bath according to claim any one of the claims 8-10, characterized in that the data processor comprises a storage module and a connecting gate for connecting the data processor to a universal data processor, for inputting a program from the universal data processor to the storage module.

12. A bath according to any one of the claims 8-11, characterized in that the pumping system (3, 4, 5, 6) is self-draining.

13. A bath according to any one of claims 8-12, characterized in that one of said reservoirs (11) contains a disinfectant and another one of said reservoirs (12) contains a decalcifier.

## Patentansprüche

1. Verfahren zum Reinigen eines Bades, mit einem Pumpsystem (3, 4, 5, 6) zum Bewirken einer kräftigen Ausströmung von Luft oder Badewasser in eine Badewanne (1), wobei Wasser und darin gelöste Reinigungsmittel durch das Pumpsystem (3, 4, 5, 6) in Umlauf gebracht werden, dadurch gekennzeichnet, daß während eines ersten Reinigungszyklus ein Entkalkungsmittel als Reinigungsmittel verwendet wird und während eines nächsten Reinigungszyklus ein Desinfektionsmittel als Reinigungsmittel verwendet wird.

2. Verfahren nach Anspruch 1, dadurch gekennzeichnet, daß die relative Frequenz der Verwendung eines Entkalkungsmittels als Reinigungsmittel von der Härte des Wassers abhängig ist.

3. Verfahren nach Anspruch 1 oder 2, dadurch gekennzeichnet, daß das Beendigen der Verwendung des Bades ermittelt wird und, ansprechend darauf, automatisch mindestens ein Reinigungszyklus durchgeführt wird.

4. Verfahren nach Anspruch 3, dadurch gekennzeichnet, daß das Dosieren der Menge an Reinigungsmittel in Abhängigkeit von in einen Datenprozessor eingespeicherten Daten gesteuert wird.

5. Verfahren nach Anspruch 4, dadurch gekennzeichnet, daß die Daten aus einem universellen Datenprozessor in den Datenprozessor eingespeichert werden.

6. Verfahren nach Anspruch 4, dadurch gekennzeichnet, daß die Daten über eine Fernsprechlinie und ein Modem in den universellen Datenprozessor eingegeben werden.

7. Verfahren nach einem der vorhergehenden Ansprüche, dadurch gekennzeichnet, daß beim Beendigen eines Reinigungszyklus das Pumpsystem (3, 4, 5, 6) im wesentlichen entleert wird.

8. Bad, mit
einer Badewanne (1),
einem Pumpsystem (3, 4, 5, 6), mit einer Pumpe (5) zum Bewirken einer kräftigen Ausströmung von Luft oder Badewasser in eine Badewanne (1),
Mitteln zum Betätigen der Pumpe (5),
einem Zuflußrohr (15), das mit einem in die Badewanne (1) mündenden Abflußrohr (26) in Verbindung steht,
mindestens zwei Behältern (11, 12), wobei einer der Behälter ein Desinfektionsmittel enthält,
Mitteln (19, 20) zum selektiven Verbinden jedes der Behälter (11, 12) mit dem Zuflußrohr (15),
mindestens einem Flüssigkeitsdosierorgan (16) zum Dosieren der von jedem der Behälter (11, 12) gelieferten Flüssigkeit,
einem Reinwasserzuflußrohr (17) mit Dosiermitteln (18),
einem Abflußkanal (9) zum Entleeren des Bades,
einem in dem Abflußkanal (9) angeordneten Abflußventil,
Mitteln zum Betätigen des Abflußventils und
einem Datenprozessor, der wirksam angeschlossen ist, um die Pumpe (5), das Flüssigkeitsdosierorgan (16) und die Mittel (19, 20) zum selektiven Verbinden jedes der Behälter (11, 12) mit dem Zuflußrohr (15) zu betätigen,
dadurch gekennzeichnet, daß
der andere der Behälter (11) ein Behälter ist, der ein Entkalkungsmittel enthält,
der Datenprozessor auch wirksam an die Dosiermittel (18) des Reinwasserzuflußrohres (17) und an die Mittel zum Betätigen des in dem Abflußkanal (9) zum Entleeren des Bades angeordneten Abflußventils angeschlossen ist und daß
der Datenprozessor zum Betätigen der Dosiermittel (18) des Reinwasserzuflußrohres (17) und der Mittel (19, 20) zum selektiven Verbinden jedes der Behälter (11, 12) mit dem Zuflußrohr (15) programmiert ist, und zwar derart, daß während eines ersten Reinigungszyklus durch das Pumpsystem (3, 4, 5, 6) in Umlauf zu bringendes Reinwasser zugeführt wird und Flüssigkeit aus einem Behälter (11) für Entkalkungsmittel mit dem genannten Wasser gemischt wird und während eines zweiten Reinigungszyklus durch das Pumpsystem (3, 4, 5, 6) in Umlauf zu bringendes Reinwasser zugeführt wird und Flüssigkeit aus einem Behälter (12) für Desinfektionsmittel mit dem genannten Wasser gemischt wird.

9. Bad nach Anspruch 8, dadurch gekennzeichnet, daß das Dosierorgan (16) in dem Zuflußrohr (15) angeordnet ist und ein Stromabwärtsende des Zuflußrohres (15) mit einem in die Badewanne (1) mündenden Abflußrohr (26) in Verbindung steht.

10. Bad nach Anspruch 8 oder 9, gekennzeichnet durch mindestens einen dritten Behälter (Pfeil 27).

11. Bad nach einem der Ansprüche 8 bis 10, dadurch gekennzeichnet, daß der Datenprozessor einen Speichermodul und ein Anschlußtor zum Anschließen des Datenprozessors an einen universellen Datenprozessor enthält, um ein Programm aus dem universellen Datenprozessor in den Speichermodul einzugeben.

12. Bad nach einem der Ansprüche 8 bis 11, dadurch gekennzeichnet, daß das Pumpsystem (3, 4, 5, 6) selbstentleerend ist.

13. Bad nach einem der Ansprüche 8 bis 12, dadurch gekennzeichnet, daß einer der Behälter (11) ein Desinfektionsmittel enthält und ein anderer der Behälter (12) ein Entkalkungsmittel enthält.

## Revendications

1. Procédé de nettoyage d'un bain comprenant un système de pompage (3, 4, 5, 6) forçant l'air ou l'eau du bain à s'écouler dans une baignoire (1), dans lequel procédé l'eau et les agents de nettoyage dissous dans celle-ci sont mis en circulation par ledit système de pompage (3, 4, 5, 6), caractérisé en ce que, pendant un premier cycle de nettoyage, un détartrant est utilisé comme agent de nettoyage et, pendant un cycle de nettoyage suivant, un désinfectant est utilisé comme agent de nettoyage.

2. Procédé selon la revendication 1, caractérisé en ce que la fréquence relative d'utilisation d'un détartrant en tant qu'agent de nettoyage dépend de la dureté de l'eau.

3. Procédé selon l'une des revendications 1 ou 2, caractérisé en ce que la fin de l'utilisation du bain est détectée et, en réaction, au moins un cycle de nettoyage est exécuté automatiquement.

4. Procédé selon la revendication 3, caractérisé en ce que le dosage de la quantité d'agent de nettoyage est commandé en fonction des données sauvées dans le processeur de données.

5. Procédé selon la revendication 4, caractérisé en ce que les données sont sauvées dans le processeur de données à partir d'un processeur universel de données.

6. Procédé selon la revendication 4, caractérisé en ce que les données sont introduites dans le processeur universel de données par l'intermédiaire d'une ligne téléphonique et d'un modem.

7. Procédé selon l'une quelconque des revendications précédentes, caractérisé en ce qu'à la fin d'un cycle de nettoyage, le système de pompage (3, 4, 5, 6) est essentiellement vidangé.

8. Bain comprenant:
une baignoire (1),
un système de pompage (3, 4, 5, 6) comprenant une pompe (5) pour forcer l'air ou l'eau du bain à s'écouler dans la baignoire (1);
des moyens pour actionner la pompe (5),
une conduite d'alimentation (15), communiquant avec une conduite d'évacuation (26) débouchant dans la baignoire (1),
au moins deux réservoirs (11, 12), un desdits réservoirs contenant un désinfectant,
des moyens (19, 20) pour raccorder sélectivement chacun desdits réservoirs (11, 12) avec la conduite d'alimentation (15),
au moins un élément de dosage de liquide (16) pour doser le liquide amené depuis chacun des réservoirs (11, 12),
une conduite d'alimentation en eau propre (17) avec des moyens de dosage (18),
un canal d'évacuation (9) pour vider le bain,
une soupape d'évacuation disposée dans ledit canal d'évacuation (9),
des moyens pour actionner ladite soupape d'évacuation, et
un processeur de données raccordé de manière opérationnelle pour actionner la pompe (5), l'élément de dosage des liquides (16) et les moyens (19, 20) pour raccorder sélectivement chacun des réservoirs (11, 12) avec la conduite d'alimentation (15),
caractérisé en ce que
l'autre desdits réservoirs (11) est un réservoir destiné à contenir un détartrant,
le processeur de données est également raccordé de manière opérationnelle aux moyens de dosage (18) de la conduite d'alimentation en eau propre (17) et aux moyens (19, 20) pour actionner ladite soupape d'évacuation disposée dans ledit canal d'évacuation (9) pour vider le bain et en ce que
le processeur de données est programmé pour actionner le moyen de dosage (18) de la conduite d'alimentation en eau propre (17) et les moyens (19, 20) pour raccorder sélectivement chacun desdits réservoirs (11, 12) avec la conduite d'alimentation (15) de telle sorte que, pendant un premier cycle de nettoyage, l'eau propre destinée à circuler à travers le système de pompage (3, 4, 5, 6) est amenée et le liquide provenant d'un réservoir (11) pour détartrant est mélangé à ladite eau et, pendant un deuxième cycle de nettoyage, de l'eau propre destinée à circuler à travers le système de pompage (3, 4, 5, 6) est amenée et le liquide d'un réservoir (12) pour désinfectant est mélangé à ladite eau.

9. Bain selon la revendication 8, caractérisé en ce que l'élément de dosage (16) est disposé dans la conduite d'alimentation (15) et une extrémité en aval de la conduite d'alimentation (15) communique avec une conduite d'évacuation (26) débouchant dans la baignoire (1).

10. Bain selon l'une des revendications 8 ou 9, caractérisé par au moins un troisième réservoir (flèche 27).

11. Bain selon l'une quelconque des revendications 8 à 10, caractérisé en ce que le processeur de données comprend un module de sauvegarde et un port de connexion pour raccorder le processeur de données à un processeur universel de données, pour introduire un programme provenant du processeur universel de données vers le module de sauvegarde.

12. Bain selon l'une quelconque des revendications 8 à 11, caractérisé en ce que le système de pompage (3, 4, 5, 6) est un système à vidange automatique.

13. Bain selon l'une quelconque des revendications 8 à 12, caractérisé en ce l'un desdits réservoirs (11) contient un désinfectant et un autre desdites réservoirs (12) contient un détartrant.
